(19) **Europäisches Patentamt / European Patent Office / Office européen des brevets**

(11) **EP 2 477 551 B1**

(12) **FASCICULE DE BREVET EUROPEEN**

(45) Date de publication et mention de la délivrance du brevet:
**14.12.2016 Bulletin 2016/50**

(21) Numéro de dépôt: **10752844.0**

(22) Date de dépôt: **17.09.2010**

(51) Int Cl.:
***A61B 8/08*** (2006.01)

(86) Numéro de dépôt international:
**PCT/EP2010/063677**

(87) Numéro de publication internationale:
**WO 2011/033050 (24.03.2011 Gazette 2011/12)**

(54) **PROCÉDÉ POUR LA MESURE D'AU MOINS UNE PROPRIÉTÉ D'UN TISSU BIOLOGIQUE**

VERFAHREN ZUR MESSUNG VON MINDESTENS EINER EIGENSCHAFT VON BIOLOGISCHEM GEWEBE

METHOD FOR MEASURING AT LEAST ONE PROPERTY OF BIOLOGICAL TISSUE

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **17.09.2009 FR 0956408**

(43) Date de publication de la demande:
**25.07.2012 Bulletin 2012/30**

(73) Titulaire: **Echosens**
**75013 Paris (FR)**

(72) Inventeurs:
• **SANDRIN, Laurent**
  **F-94240 L'Hay les Roses (FR)**
• **MIETTE, Véronique**
  **F-94800 Villejuif (FR)**
• **SASSO, Magali**
  **F-75012 Paris (FR)**

(74) Mandataire: **Lebkiri, Alexandre**
**Cabinet Camus Lebkiri**
**25, Rue de Maubeuge**
**75009 Paris (FR)**

(56) Documents cités:
EP-A1- 1 629 777      WO-A1-2007/100107
WO-A1-2008/139245    WO-A1-2009/107673
US-A- 5 906 578      US-A- 6 013 031
US-A1- 2002 068 870

EP 2 477 551 B1

**Description**

L'invention porte sur un procédé pour la mesure d'au moins une propriété de tissu biologique. Elle trouve une application particulière dans le domaine humain ou animal.

De façon connue, pour mesurer les propriétés viscoélastiques de tissu biologique, il est fréquent de mesurer ces dernières au moyen de l'élastographie impulsionnelle comme décrit, par exemple, dans la demande de brevet numéro FR 2843290. Le procédé divulgué par ce document consiste à positionner une sonde, comportant un transducteur ultrasonore et un générateur de vibration basse fréquence, au contact de l'épiderme et plus particulièrement en regard du tissu biologique à mesurer. Une onde élastique basse fréquence est alors générée au moyen du générateur de vibration basse fréquence dans le tissu biologique. Parallèlement, des émissions et acquisitions ultrasonores sont réalisées au moyen du transducteur ultrasonore au cours de la propagation de l'onde élastique basse fréquence pour observer le déplacement du tissu biologique soumis à l'onde élastique basse fréquence. Des valeurs sont ensuite calculées en fonction de ce déplacement.

Le document US6013031A1 divulgue un procédé différent pour la mesure d'au moins une propriété.

Un inconvénient de cet état de la technique réside en ce que l'opérateur ne peut affirmer avec certitude que le transducteur ultrasonore soit positionné en regard du tissu à mesurer. En conséquence, les valeurs obtenues par la mise en oeuvre d'un tel procédé peuvent ne pas être représentatives du tissu que l'opérateur cherche à mesurer.

Comme les valeurs dépendent particulièrement de la position de la sonde, laquelle est positionnée de façon approximative par l'opérateur en fonction de ses connaissances, l'habilité et les connaissances de l'opérateur influent en grande partie sur les valeurs obtenues. Ces dernières sont donc très dépendantes de l'opérateur et la mise en oeuvre d'un tel procédé impose à l'opérateur de posséder des connaissances accrues dans le domaine humain ou animal.

L'invention a donc plus particulièrement pour but de remédier aux inconvénients du procédé précité. Dans ce contexte, l'invention vise à proposer un procédé pour la mesure de propriétés de tissu biologique correspondant effectivement au tissu que l'opérateur souhaite mesurer. De surcroît, l'invention vise également à proposer un procédé pour la mesure de propriétés de tissu biologique ne nécessitant pas pour l'opérateur de connaissances accrues dans le domaine humain ou animal.

A cette fin, l'invention porte sur un procédé selon la revendication 1.

On désigne pour la suite de la description par paramètre, une valeur mesurable ou une combinaison de valeurs mesurables pouvant correspondre à une caractéristique physique, physiologique, viscoélastique, ultrasonore ou toute autre caractéristique d'un milieu tel que par exemple un tissu biologique.

Dans le cadre de l'invention, le paramètre est destiné à valider l'hypothèse de présence du tissu cible en regard du transducteur ultrasonore par comparaison avec un paramètre de référence du tissu biologique cible pouvant être, de façon non limitative, une valeur de référence, une gamme de valeur de référence, un abaque de référence ou un gabarit de référence déterminés empiriquement. Par ailleurs, la comparaison peut consister à déterminer un phénomène physiologique tel que par exemple la détection ou l'absence de détection d'une onde de cisaillement se propageant dans le tissu biologique.

On désigne pour la suite de la description par propriété une valeur représentative d'une caractéristique intrinsèque d'un milieu tel que par exemple les tissus biologiques. Cette propriété peut être issue d'une mesure ou déterminée par un modèle de type physique ou physiologique décrit par une série de paramètres.

Selon la définition précédente, le paramètre peut être formé par la fréquence centrale de signal ultrasonore rétrodiffusé par les tissus biologiques. La propriété associée peut être par exemple l'atténuation ultrasonore qui est reliée par un modèle physique à la diminution de la fréquence centrale dans les tissus biologiques.

De façon générale, une valeur mesurée peut être à la fois un paramètre ou une propriété. A titre illustratif, le poids est à la fois une valeur mesurée par une balance (paramètre) et une propriété d'un corps.

Grâce à l'invention, la présence d'un tissu biologique cible est vérifiée avant qu'au moins une propriété du tissu biologique ne soit déterminée ou est vérifiée simultanément à la détermination d'au moins une propriété du tissu biologique à la condition qu'au moins un paramètre déterminé corresponde à au moins un paramètre de référence correspondant. En conséquence, les propriétés déterminées par le procédé de l'invention correspondent effectivement aux propriétés du tissu biologique souhaité. Cette particularité confère à ce procédé une facilité d'utilisation pour l'opérateur et l'opérateur ne doit pas obligatoirement posséder de fortes connaissances dans le domaine humain ou animal pour la mise en oeuvre du procédé conforme à l'invention.

Outre les caractéristiques principales qui viennent d'être mentionnées dans le paragraphe précédent, le procédé pour la mesure d'au moins une propriété de tissu biologique selon l'invention peut présenter une ou plusieurs caractéristiques supplémentaires ci-dessous, considérées individuellement ou selon toutes les combinaisons techniquement possibles :

- une pluralité de paramètres sont combinés entre eux, ladite étape de comparaison consistant à comparer un résultat issu de ladite combinaison de paramètres audit au moins un paramètre de référence ;

- ledit procédé comporte une étape de génération d'une onde élastique basse fréquence dans ledit tissu biologique ;
- ladite onde élastique basse fréquence est générée par vibration d'un générateur d'onde élastique basse fréquence ;
- ladite onde élastique basse fréquence est générée par pression de radiation ;
- un indicateur informe un opérateur dudit résultat de ladite étape de comparaison ; l'indicateur pouvant être un indicateur visuel situé sur la sonde ou sur un écran apte à communiquer avec la sonde ou encore un indicateur sonore.
- ledit paramètre et ladite propriété dudit tissu biologique sont déterminés en fonction de données extraites de ladite acquisition dudit au moins un signal ultrasonore réfléchi par le tissu biologique ; grâce à cette particularité, une unique mesure est réalisée pour déterminer d'une part un paramètre représentatif dudit tissu biologique et d'autre part une propriété dudit tissu biologique ;
- ladite au moins une propriété dudit tissu biologique est déterminée par la mise en oeuvre d'un procédé d'élastographie ;
- ladite au moins une propriété est l'élasticité dudit tissu biologique ;
- ladite au moins une propriété est une atténuation ultrasonore dudit tissu biologique ;
- ledit au moins un paramètre est un paramètre ultrasonore dudit tissu biologique ;
- ledit au moins un paramètre ultrasonore est une atténuation ultrasonore dudit tissu biologique ;
- ledit au moins un paramètre est un paramètre viscoélastique dudit tissu biologique ;
- ledit au moins un paramètre viscoélastique est l'élasticité dudit tissu biologique ;
- ladite élasticité est obtenue par un procédé d'élastographie vibratoire ;
- ledit au moins un paramètre est un paramètre physiologique dudit tissu biologique.

D'autres caractéristiques et avantages de l'invention ressortiront clairement de la description qui en est donnée ci-après, à titre indicatif et nullement limitatif, en référence aux figures annexées ci-jointes parmi lesquelles :

- la figure 1 représente un organigramme du principe de fonctionnement d'un procédé conforme à l'invention ;
- la figure 2 représente un exemple de mise en oeuvre possible du procédé de l'invention ;
- la figure 3 représente un exemple de transducteur ultrasonore conforme à l'invention.

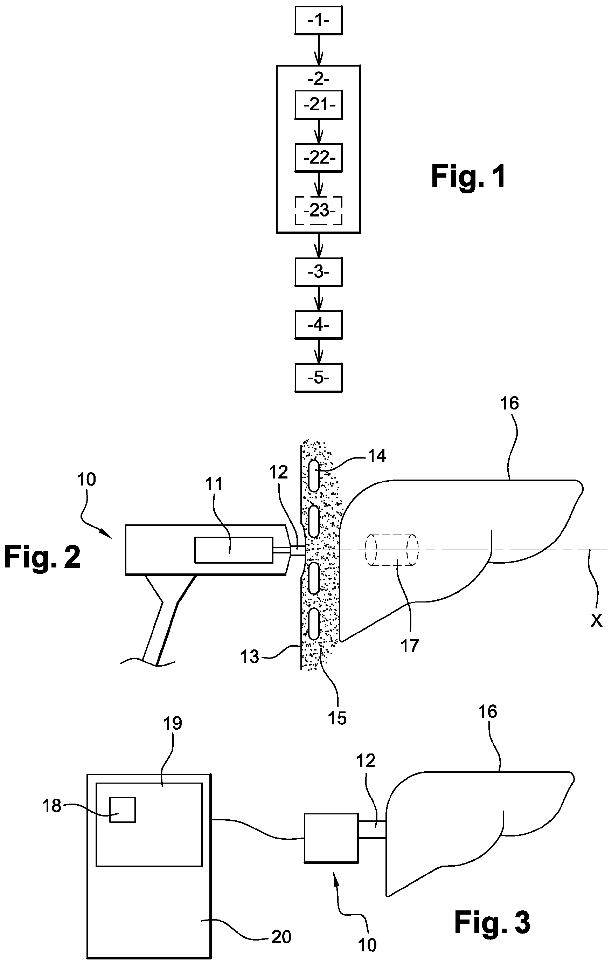

Pour des raisons de clarté, seuls les éléments utiles pour la compréhension de l'invention ont été représentés, et ceci sans respect de l'échelle et de manière schématique.
Il convient de noter qu'une onde élastique basse fréquence peut être comprise, par exemple, entre 10 et 1000 Hz.
Notons qu'une onde ultrasonore peut être comprise, par exemple, entre 20 KHz et 1000 MHz.
Dans un exemple non limitatif, le tissu biologique utilisé dans la suite de la description pour illustrer la mise en oeuvre du procédé conforme à l'invention est le foie.
Les étapes nécessaires pour la réalisation du procédé pour la mesure d'au moins une propriété de tissu biologique sont à présent décrites à l'appui des figures 1 à 3.
Selon une première étape 1, on positionne un transducteur ultrasonore en regard d'un tissu biologique.
On notera que dans des modes de réalisation non limitatifs, différents types de transducteurs ultrasonores monoélément ou multiéléments peuvent être utilisés. Il peut s'agir d'un transducteur de type couronne, annulaire, matrice 2D, barrette linéaire ou convexe, d'un transducteur de type étoilé ou tout autre type de transducteur apte à émettre et recevoir des signaux ultrasonores.
Dans l'exemple du foie, une sonde ultrasonore, comportant au moins le transducteur ultrasonore apte à émettre et à recevoir des signaux ultrasonores, est positionnée au contact de l'épiderme et en regard du foie de façon à ce que les signaux ultrasonores émis par la sonde ultrasonore puissent se propager au sein du foie.
Selon une deuxième étape 2, on mesure au moins un paramètre du tissu biologique, la mesure comportant une première sous étape 21 et une deuxième sous étape 22.
Lors de la première sous-étape 21, au moins un signal ultrasonore est généré par le transducteur ultrasonore au sein du tissu biologique.
Lors de la deuxième sous-étape 22, une acquisition d'au moins un signal ultrasonore réfléchi par le tissu biologique est réalisée.
Dans l'exemple du foie, la mesure d'au moins un paramètre est effectuée dans une région d'intérêt (ROI pour Region Of Interest en anglais). La région d'intérêt est par exemple située entre 25 et 65 mm sous l'épiderme. La mesure d'un paramètre du tissu de la région d'intérêt peut, par exemple, être effectuée au moyen d'un procédé d'élastographie vibratoire mettant en oeuvre une sonde d'élastographie vibratoire tel qu'illustré sur la figure 2.
Ainsi, dans une telle mise en oeuvre, une troisième sous-étape 23 supplémentaire est nécessaire. Cette troisième sous-étape 23 consiste à générer une onde élastique basse fréquence dans le foie. La propagation de cette onde élastique basse fréquence étant par exemple, suivie de la génération du signal ultrasonore (première sous-étape 21) et de l'acquisition du signal ultrasonore réfléchi par le foie (deuxième sous-étape 22).
L'ordre des sous-étapes 21, 22 et 23 est donné ici à titre d'exemple. De ce fait, l'ordre peut être différent.

La figure 2 représente :

- une sonde 10 d'élastographie vibratoire munie d'un générateur 11 d'ondes élastiques basse fréquence et d'un transducteur ultrasonore 12 ;
- l'épiderme 13 ;
- quatre côtes 14 ;
- un tissu sous-cutané 15 ;
- un tissu biologique formé, de façon non limitative, par un foie 16 ;
- une région d'intérêt 17 ;
- un axe X d'émissions ultrasonores.

Dans ce mode de réalisation, la sonde positionnée au cours de la première étape 1 est la sonde 10 d'élastographie vibratoire. En vue de déterminer un paramètre viscoélastique du foie 16, le transducteur ultrasonore 12 de la sonde 10 est positionné, au cours de la première étape 1, au contact de l'épiderme 13 et dans l'espace intercostal, autrement dit entre deux des quatre côtes 14. Le générateur 11 d'ondes élastiques basse fréquence génère, par contact indirect avec le foie 16, une ou plusieurs onde(s) élastique(s) basse fréquence se propageant dans le tissu sous-cutané 15, puis dans le foie 16. Cette (ces) onde(s) élastique(s) basse fréquence est (sont) généralement obtenue(s) de manière mécanique mais peut (peuvent) également être obtenue(s) par pression de radiation, par hyperthermie ultrasonore ou encore par des vibrations internes du corps (battements cardiaque, pouls, etc.). La forme temporelle de cette (ces) onde(s) élastique(s) basse fréquence peut être arbitraire, mais plus généralement de type impulsionnelle, transitoire ou périodique (entretenue, monochromatique).

Dans le même temps, des ondes ultrasonores sont générées et acquises le long de l'axe X au moyen du transducteur ultrasonore 12 pour suivre la propagation de cette (ces) onde(s) élastique(s) basse fréquence au sein de la région d'intérêt 17, à savoir entre 25 et 65 mm sous l'épiderme 13.

Selon une troisième étape 3, on détermine au moins un paramètre du tissu biologique au moyen de l'acquisition d'au moins un signal ultrasonore réfléchi par le tissu biologique effectuée au cours de la deuxième sous-étape 22. Le ou les paramètre(s) est (sont) représentatif(s) du tissu biologique.

Selon des variantes avantageuses du procédé conforme à l'invention, un paramètre recherché peut être un paramètre viscoélastique, un paramètre ultrasonore ou un paramètre physiologique.

- En ce qui concerne le paramètre viscoélastique, on désigne de façon non limitative par paramètre viscoélastique d'un tissu biologique, au moins une propriété mécanique décrivant le comportement viscoélastique de ce tissu biologique. La propriété mécanique peut être, par exemple, formée par un module d'Young, un module de cisaillement, des propriétés des ondes se propageant dans le tissu biologique viscoélastique telles qu'une vitesse ultrasonore, une dispersion en vitesse ultrasonore, une atténuation d'une onde élastique basse fréquence ou encore des paramètres associés à un modèle viscoélastique du tissu biologique tel que par exemple le modèle de Maxwell, le modèle de Voigt ou le modèle de Zener.

Dans l'exemple du foie 16 vu précédemment, des paramètres viscoélastiques sont déterminés.

Un premier paramètre viscoélastique correspond, par exemple, à la détection d'une onde élastique basse fréquence dans la région d'intérêt 17.

Un deuxième paramètre viscoélastique correspond, par exemple, à la vitesse de déplacement de cette onde élastique basse fréquence au sein de la région d'intérêt 17.

Un troisième paramètre viscoélastique correspond, par exemple, à une valeur d'élasticité du tissu biologique de la région d'intérêt 17.

Ainsi, trois paramètres viscoélastiques peuvent être déduits de la mesure effectuée lors de la deuxième étape 2.

En outre, une mesure d'un paramètre du tissu biologique se trouvant au sein de la région d'intérêt 17 peut être effectuée au moyen d'un procédé différent de l'élastographie vibratoire. A titre d'exemple, des paramètres peuvent être déterminés à partir de données extraites du signal ultrasonore réfléchi par le tissu biologique de la région d'intérêt 17. De ce fait, une simple émission et acquisition ultrasonore au moyen du transducteur ultrasonore 12 suffisent pour la mesure d'un paramètre relatif au tissu biologique (16) que comporte la région d'intérêt 17.

- En ce qui concerne le paramètre ultrasonore, on désigne de façon non limitative par paramètre ultrasonore d'un tissu biologique, une vitesse ultrasonore, une mesure de dispersion en vitesse ultrasonore, une atténuation ultrasonore ou encore un coefficient de rétrodiffusion ultrasonore.

En outre, dans le domaine temporel le paramètre ultrasonore peut être formé par exemple par une puissance du signal ultrasonore, une énergie du signal ultrasonore, un coefficient de corrélation ou d'inter-corrélation.

Dans le domaine spectral le paramètre ultrasonore peut être formé par exemple par un décalage de la fréquence centrale du signal ultrasonore reçu par rapport à la fréquence centrale du signal ultrasonore émis.

Par ailleurs, le paramètre ultrasonore peut être obtenu dans des domaines transformés comme par exemple le domaine temps-fréquence ou le domaine cepstral.

Il est entendu que les paramètres ultrasonores précités sont donnés ici à titre purement illustratif et sont de fait non exhaustifs.

- En ce qui concerne le paramètre physiologique, on désigne de façon non limitative par paramètre physiologique d'un tissu biologique, la détection d'un flux sanguin traversant le tissu biologique ou la fréquence organique du tissu biologique.

Comme précédemment indiqué, le paramètre physiologique peut être formé par la détection d'un flux sanguin traversant le tissu biologique.

A titre d'exemple, une échographie Doppler peut être réalisée pour déterminer si la région d'intérêt du tissu biologique comporte un flux sanguin et donc une veine.

Selon une quatrième étape 4, on compare au moins un paramètre du tissu biologique à au moins un paramètre de référence d'un tissu biologique cible.

En d'autres termes, des moyens logiciels non représentés vérifient de façon automatique que le ou les paramètre(s) déterminé(s) lors de la troisième étape 3 possède des caractéristiques sensiblement similaires aux caractéristiques de référence que comporte le tissu biologique cible. Pour ce faire, une comparaison entre le ou les paramètre(s) de référence viscoélastique(s) et/ou ultrasonore(s) et/ou physiologique(s) du tissu cible (le foie 16 dans notre exemple) et le ou les paramètre(s) viscoélastique(s) et/ou ultrasonore(s) et/ou physiologique(s) du tissu mesuré est réalisée.

A titre d'exemple, lorsque le paramètre du tissu biologique mesuré diffère du paramètre de référence (pouvant être formé par une gamme de valeurs) du tissu biologique cible, un indicateur informe un opérateur du résultat de ladite comparaison. Ainsi, l'opérateur est, par exemple, invité par un indicateur visuel 18 s'affichant sur un écran 19 que comporte un dispositif 20 (représenté à la figure 3), lequel dispositif 20 est relié à la sonde 10 comportant le transducteur ultrasonore 12, à déplacer la sonde 10 de façon à ce que le transducteur ultrasonore 12 se trouve en regard du tissu biologique que souhaite mesurer l'opérateur. Le graphisme de l'indicateur visuel 18 étant fonction du paramètre déterminé. Le graphisme de l'indicateur visuel 18 pouvant, de façon non limitative, être formé par un voyant lumineux affichant :

- une couleur verte lorsque le paramètre déterminé correspond au paramètre de référence ;
- une couleur rouge lorsque le paramètre déterminé diffère du paramètre de référence.

Selon une telle mise en oeuvre, lorsque l'indicateur visuel 18 affiche une couleur rouge, cela indique à l'opérateur que le transducteur ultrasonore 12 n'est pas situé en regard du tissu biologique dont il souhaite déterminer une propriété.

Les étapes 1 à 4 sont ainsi réitérées jusqu'à ce que le paramètre du tissu biologique se trouvant en regard du transducteur ultrasonore 12 corresponde sensiblement au paramètre de référence du tissu biologique cible. Cette condition pouvant, par exemple, être indiquée à l'opérateur au moyen de l'indicateur visuel 18 affichant une couleur verte.

Par ailleurs, l'indicateur visuel peut être affiché par une LED (non représentée) que comporte la sonde 10 comportant le transducteur ultrasonore 12. Cette LED changeant de couleur en fonction du résultat de la comparaison du paramètre déterminé avec le paramètre de référence.

En outre, l'indicateur peut être formé par un indicateur sonore.

L'indicateur peut être formé par tout autre moyen informant l'opérateur du résultat de la comparaison d'un ou de plusieurs paramètre(s) déterminé(s) avec un ou plusieurs paramètre(s) de référence.

Selon une mise en oeuvre différente, lorsqu'un paramètre déterminé diffère du paramètre de référence correspondant du tissu biologique cible, l'opérateur ne peut pas déterminer une propriété. Cette impossibilité n'est pas obligatoirement indiquée au moyen d'un indicateur. Par exemple, l'absence d'affichage d'une valeur représentative d'une propriété obtenue ultérieurement (lors de la cinquième étape 5) peut informer l'opérateur que le transducteur ultrasonore n'est pas situé en regard du tissu à mesurer.

Dans l'exemple du foie 16 vu précédemment, la présence de la propagation d'une onde élastique basse fréquence générée au cours de la deuxième étape 2 peut être utilisée comme paramètre viscoélastique du tissu biologique.

A ce titre, il convient de noter que le transducteur ultrasonore 12 de la sonde 10 est positionné entre deux des quatre côtes 14. De ce fait, cet emplacement interdit l'accès à certain organes comme par exemple la thyroïde. Toutefois, cet emplacement peut donner accès aux poumons, aux intestins et éventuellement aux reins.

En ce qui concerne l'accès aux poumons, il est important de noter que les ondes ultrasonores ne se propagent pas dans l'air. Dans l'hypothèse selon laquelle une onde élastique basse fréquence est détectée au moyen d'un signal ultrasonore réfléchi par le foie 16, cette caractéristique exclue la possibilité que l'onde élastique basse fréquence ait été générée dans les poumons.

En ce qui concerne l'accès aux intestins, il est connu que la faible épaisseur de la paroi des intestins ne permette pas la détection d'une onde élastique basse fréquence.

En ce qui concerne l'accès aux reins, Il est probable qu'une onde élastique basse fréquence puisse se propager dans les reins. En revanche, puisque la propagation de l'onde élastique basse fréquence est suivie sur une profondeur comprise entre 25 et 65 mm, on exclut la possibilité de détection d'une onde élastique basse fréquence dans les reins.

En résumé, pour une application non limitative du procédé conforme à l'invention, le positionnement de la sonde 10 d'élastographie vibratoire entre deux des quatre côtes 14 et la détection de la propagation d'une onde élastique basse fréquence (paramètre du foie 16) dans la région d'intérêt 17 formé sur une hauteur comprise entre 25 et 65 mm permet d'assurer que la sonde 10 d'élastographie vibratoire est positionnée correctement pour déterminé au moins une propriété du foie 16.

En outre, comme vu précédemment le paramètre du foie 16 peut être formé par un paramètre physiologique, tel que par exemple la détection d'un flux sanguin au moyen d'une échographie Doppler. Dans une telle mise en oeuvre, si le flux sanguin correspond à un flux sanguin de référence alors la sonde 10 d'élastographie vibratoire est correctement positionnée pour déterminer une propriété du foie 16.

De façon générale, il est entendu que tout paramètre représentatif du tissu biologique peut être utilisé pour valider le positionnement du transducteur ultrasonore 12. De même une pluralité de paramètres peut être utilisée pour valider le positionnement du transducteur ultrasonore 12.

En outre, plusieurs paramètres peuvent être combinés pour valider la présence d'un tissu cible en regard du transducteur ultrasonore 12. Ainsi, selon une telle mise en oeuvre, un résultat issu de la combinaison de ces paramètres est comparé à un paramètre de référence, la détermination d'une propriété (cinquième étape 5) se faisant uniquement si le résultat issu de la combinaison est sensiblement similaire à la valeur du paramètre de référence. Les paramètres peuvent être combinés suivant différents modèles comme, par exemple, par un modèle de type régression logistique. La régression logistique permet de construire un modèle de prédiction incluant les valeurs des paramètres déterminés.

A titre d'exemple, la valeur issue de la combinaison peut être obtenue au moyen d'une formule de ce type :

$$\text{Valeur de prédiction (résultat issu de la combinaison) } = a+b^{*} \text{Parametre\_1[...]}+c^{*}\text{Parametre\_2[...]...}$$

Il est à noter que le symbole « [...] » représente un vecteur de valeurs formant un paramètre déterminé.

Les termes a, b et c sont des constantes.

Selon une cinquième étape 5, on détermine une propriété du tissu biologique en fonction du résultat obtenu lors de la quatrième étape 4 de comparaison.

Par exemple, si la valeur absolue de la différence entre la valeur d'un paramètre déterminée au cours de la troisième étape 3 et la valeur du paramètre de référence correspondant est inférieure à une valeur seuil donnée alors on détermine une propriété du tissu biologique.

En outre, selon une mise en oeuvre différente, le paramètre peut être formé par des valeurs liées à la propagation d'une onde élastique basse fréquence. Ces valeurs peuvent par exemple correspondre à des niveaux d'amplitude ou des critères de qualité d'image. Selon une telle mise en oeuvre, le résultat obtenu lors de la quatrième étape 4 est formé par « la détection d'une onde élastique basse fréquence ». Si cette onde élastique basse fréquence est détectée alors on détermine une propriété du tissu biologique.

Pour ce faire, la propriété du tissu biologique peut être déterminée par la mise en oeuvre d'un procédé.

Le procédé peut être, de façon non limitative, un procédé d'élastographie vibratoire mis en oeuvre au moyen d'une sonde 10 d'élastographie vibratoire conforme à celui mis en oeuvre lors de la deuxième étape 2 ou un procédé nécessitant de simples émissions et acquisitions ultrasonores au moyen d'un transducteur ultrasonore 12. Cette dernière mise en oeuvre peut être réalisée en vue de déterminer une atténuation ultrasonore générée par le tissu biologique.

La détermination d'une propriété peut être déclenchée de façon automatique par les moyens logiciels (non représentés) si la valeur absolue de la différence entre la valeur du paramètre déterminée au cours de la troisième étape 3 et la valeur du paramètre de référence est inférieure à une valeur seuil donnée. En d'autres termes, aucune action, aucun déclenchement pour déterminer une propriété n'est effectué par l'opérateur.

De façon différente, la détermination d'une propriété peut être déclenchée de façon manuelle par appui de l'opérateur, après que ce dernier ait reçu un message sonore et/ou visuel (au moyen d'un indicateur) lui indiquant que la sonde 10 est positionnée correctement, sur un bouton déclencheur (non représenté) que peut éventuellement comporter la sonde 10.

Pour mesurer l'atténuation ultrasonore de multiples algorithmes sont décrit dans la littérature. A titre d'exemple et de façon non limitative on peut citer l'algorithme d'évaluation de l'atténuation ultrasonore dénommé « Fréquency-shift method » ou encore « Zero-crossing method ». Le principe de cet algorithme est décrit dans le document numéro US 4441368.

De façon générale, la fréquence centrale d'un signal ultrasonore diminue lorsqu'elle traverse les tissus biologiques. En conséquence, une évaluation d'un décalage de la fréquence centrale du signal ultrasonore permet d'évaluer l'atténuation ultrasonore. La fréquence centrale du signal ultrasonore peut être estimée dans le domaine temporel en comptant le nombre de passages par zéro dans une fenêtre temporelle.

De façon non limitative, un algorithme de calcul de l'atténuation ultrasonore peut comporter les étapes suivantes :

- sélectionner une région d'intérêt 17 pour chaque signal ultrasonore radiofréquence (dans l'exemple du foie 16, la région d'intérêt 17 peut être par exemple située entre 25 et 65 mm sous l'épiderme 13) ;
- réaliser un fenêtrage temporel de chaque signal ultrasonore radiofréquence de la région d'intérêt 17, le nombre de fenêtres pouvant variées de 1 à n pour une même durée T, chaque fenêtre étant recouverte de la fenêtre voisine d'un pourcentage déterminé ;
- évaluer, sur chaque fenêtre, le nombre de passage du signal ultrasonore radiofréquence par zéro ; (ainsi, plus le signal ultrasonore radiofréquence est atténué et plus le nombre de passage par zéro sur une fenêtre de durée T va diminuer) ;
- déterminer une valeur d'atténuation ultrasonore au moyen d'une formule mathématique.

Il convient de noter que l'atténuation ultrasonore d'un même tissu biologique est différente si ce dernier possède une faible proportion de tissu adipeux ou une importante proportion de tissu adipeux car l'impédance de la graisse est différente de celle des tissus mous. Outre le fait d'être représentatif d'un tissu biologique cible, l'atténuation ultrasonore peut permettre de déterminer de façon quantitative et/ou qualitative une proportion de graisse que peut comporter le tissu biologique mesuré, comme par exemple le foie 16 dans notre exemple.

De façon différente, lors de la cinquième étape 5, la propriété du tissu biologique peut être déterminée en fonction de données extraites de l'acquisition du au moins un signal ultrasonore réfléchi par le tissu biologique réalisée au cours de la deuxième étape 2. Cette possibilité est particulièrement avantageuse car elle nécessite une unique mesure pour réaliser à la fois la troisième étape 3 (déterminer un paramètre du tissu biologique au moyen de l'acquisition du au moins un signal ultrasonore réfléchi par le tissu biologique) et la cinquième étape 5 (déterminer une propriété du tissu biologique en fonction du résultat de la comparaison). Par conséquent, la position de la sonde 10 n'a pas été modifiée et l'opérateur peut affirmer avec certitude que la propriété obtenue par la mise en oeuvre du procédé de l'invention correspond à une propriété du tissu biologique qu'il souhaite mesurer. Dans l'exemple du foie 16 illustré au cours de la description, la détection de la propagation d'une onde élastique basse fréquence dans le tissu biologique de la région d'intérêt 17 peut être utilisée comme paramètre viscoélastique servant à vérifier que le tissu mesuré corresponde au tissu cible et une fois cette hypothèse validée, la détermination de la vitesse de propagation de l'onde élastique basse fréquence permet d'estimer une propriété du tissu biologique mesuré, à savoir l'élasticité. Une unique mesure est donc nécessaire.

Grâce à l'invention, la mesure d'au moins un paramètre du tissu biologique peut permettre de déterminer de façon quantitative et/ou qualitative l'élasticité du tissu biologique et/ou l'atténuation ultrasonore d'un tissu biologique.

De façon générale, lorsqu'un paramètre viscoélastique et/ou ultrasonore et/ou physiologique du tissu biologique determiné diffère du paramètre viscoélastique et/ou ultrasonore et/ou physiologique de référence du tissu cible correspondant, aucune propriété ne peut être déterminée. Ainsi, la valeur caractéristique de la propriété obtenue est représentative du tissu biologique que souhaite mesurer l'opérateur. Aucune connaissance spécifique n'est donc requise pour le positionnement de la sonde en regard du tissu biologique. En outre, le positionnement de la sonde et donc du transducteur ultrasonore en regard du tissu biologique cible peut être par exemple précisé à l'opérateur au moyen d'un indicateur.

En d'autres termes, le procédé pour la mesure d'au moins une propriété d'un tissu biologique conforme à l'invention, offre la possibilité à un opérateur ne détenant pas de connaissances spécifiques dans le domaine humain ou animal, de réaliser des mesures de propriété d'un tissu biologique cible de façon à déterminer par exemple l'élasticité du tissu biologique cible et/ou l'atténuation ultrasonore du tissu biologique cible.

Par ailleurs, l'invention a été plus particulièrement décrite pour une application à un organe, le foie 16. Toutefois, il peut également être souhaitable d'appliquer le même procédé dans le cas de tout type d'organe humain ou animal tel que par exemple un sein, un tissu adipeux, une glande, un ganglion et, ce de façon in-vivo ou in-vitro ou encore pour effectuer un contrôle qualité pour des applications industrielles, notamment agroalimentaires.

L'invention est décrite dans ce qui précède à titre d'exemple, il est entendu que l'homme du métier est à même de réaliser différentes variantes du procédé pour la mesure d'au moins une propriété d'un tissu biologique, en particulier concernant les paramètres sans pour autant sortir du cadre du brevet.

**Revendications**

1. Procédé pour la mesure d'au moins une propriété d'un tissu biologique (12) comportant les étapes de :

   - positionner (1) un transducteur ultrasonore (12) en regard dudit tissu biologique (16) à mesurer ;
   - générer (21) au moins un signal ultrasonore au sein dudit tissu biologique (16) ;
   - acquérir (22) au moins un signal ultrasonore réfléchi par ledit tissu biologique (16) ;
   - déterminer (3) au moins un paramètre dudit tissu biologique (16) au moyen de ladite acquisition (22) dudit au moins un signal ultrasonore réfléchi par ledit tissu biologique (16), ledit au moins un paramètre étant représentatif dudit tissu biologique (16) ;

   ledit procédé étant **caractérisé en ce qu'**il comporte en outre les étapes de :

   - comparer (4) ledit au moins un paramètre dudit tissu biologique (16) à au moins un paramètre de référence d'un tissu biologique cible de façon à valider l'hypothèse de présence dudit tissu biologique cible en regard dudit transducteur ultrasonore ; ladite comparaison (4) consistant à comparer une valeur dudit au moins un paramètre déterminé à une valeur dudit au moins un paramètre de référence,
   - déterminer (5) au moins une propriété dudit tissu biologique (16) en fonction du résultat de ladite étape de comparaison (4), ladite étape de détermination (5) de ladite au moins une propriété se faisant uniquement si la valeur absolue de la différence entre ladite valeur dudit au moins un paramètre déterminé et ladite valeur dudit au moins un paramètre de référence est inférieure à une valeur seuil donnée.

2. Procédé selon la revendication 1 **caractérisé en ce qu'**une pluralité de paramètres sont combinés entre eux, ladite étape de comparaison (4) consistant à comparer un résultat issu de ladite combinaison de paramètres audit au moins un paramètre de référence.

3. Procédé selon l'une des revendications 1 ou 2 **caractérisé en ce qu'**il comporte une étape de génération (23) d'une onde élastique basse fréquence dans ledit tissu biologique (16).

4. Procédé selon la revendication 3 **caractérisé en ce que** ladite onde élastique basse fréquence est générée par vibration d'un générateur (11) d'onde élastique basse fréquence.

5. Procédé selon la revendication 3 **caractérisé en ce que** ladite onde élastique basse fréquence est générée par pression de radiation.

6. Procédé selon l'une des revendications 1 à 5 **caractérisé en ce qu'**un indicateur informe un opérateur dudit résultat de ladite étape de comparaison (4).

7. Procédé selon l'une des revendications 1 à 6 **caractérisé en ce que** ledit au moins un paramètre et ladite au moins une propriété dudit tissu biologique (16) sont déterminés en fonction de données extraites de ladite acquisition (22) dudit au moins un signal ultrasonore réfléchi par le tissu biologique (16).

8. Procédé selon l'une des revendications 1 à 7 **caractérisé en ce que** ladite au moins une propriété dudit tissu biologique (16) est déterminée par la mise en oeuvre d'un procédé d'élastographie.

9. Procédé selon l'une des revendications 1 à 8 **caractérisé en ce que** ladite au moins une propriété est l'élasticité dudit tissu biologique (16).

10. Procédé selon l'une des revendications 1 à 7 **caractérisé en ce que** ladite au moins une propriété est une atténuation ultrasonore dudit tissu biologique (16).

11. Procédé selon l'une des revendications 1 à 10 **caractérisé en ce que** ledit au moins un paramètre est un paramètre ultrasonore dudit tissu biologique (16).

12. Procédé selon la revendication 11 **caractérisé en ce que** ledit au moins un paramètre ultrasonore est une atténuation ultrasonore dudit tissu biologique (16).

13. Procédé selon l'une des revendications 1 à 10 **caractérisé en ce que** ledit au moins un paramètre est un paramètre viscoélastique dudit tissu biologique (16).

**14.** Procédé selon la revendication 13 **caractérisé en ce que** ledit paramètre viscoélastique est l'élasticité dudit tissu biologique (16).

**15.** Procédé selon la revendication 14 **caractérisé en ce que** ladite élasticité est obtenue par un procédé d'élastographie.

**16.** Procédé selon l'une des revendications 1 à 10 **caractérisé en ce que** ledit au moins un paramètre est un paramètre physiologique dudit tissu biologique (16).

**Patentansprüche**

**1.** Verfahren für die Messung wenigstens einer Eigenschaft eines biologischen Gewebes (12), umfassend die Schritte:

- Positionierung (1) eines Ultraschalltransduktors (12) gegenüber dem genannten, zu messenden biologischen Gewebe (16);
- Generierung (21) wenigstens eines Ultraschallsignals innerhalb des genannten biologischen Gewebes (16);
- Erwerb (22) wenigstens eines von dem genannten biologischen Gewebe (16) reflektierten Ultraschallsignals;
- Bestimmung (3) wenigstens eines Parameters des genannten biologischen Gewebes (16) mittels des genannten Erwerbs (22) des genannten wenigstens einen Ultraschallsignals, das durch das genannte biologische Gewebe (16) reflektiert wird, wobei der genannte wenigstens eine Parameter für das genannte biologische Gewebe (16) repräsentativ ist;

wobei das genannte Verfahren **dadurch gekennzeichnet ist, dass** es darüber hinaus die Schritte umfasst:

- Vergleich (4) des genannten wenigstens einen Parameters des genannten biologischen Gewebes (16) mit wenigstens einem Referenzparameter eines biologischen Zielgewebes derart, dass die Hypothese der Präsenz des genannten biologischen Gewebes gegenüber dem Ultraschalltransduktor validiert wird; wobei der genannte Vergleich (4) in dem Vergleich eines Wertes des genannten wenigstens einen Referenzparameters besteht, der auf einen Wert des genannten wenigstens einen Referenzparameters bestimmt wird,
- Bestimmung (5) wenigstens einer Eigenschaft des genannten biologischen Gewebes (16) in Abhängigkeit von dem Ergebnis des genannten Vergleichsschritts (4), wobei der genannte Bestimmungsschritt (5) der genannten wenigstens einen Eigenschaft nur dann erfolgt, wenn der absolute Wert der Differenz zwischen dem genannten Wert des genannten wenigstens einen bestimmten Parameters und dem genannten Wert des genannten wenigsten einen Referenzparameters geringer ist als ein bestimmter Schwellenwert.

**2.** Verfahren gemäß Anspruch 1, **dadurch gekennzeichnet, dass** eine Vielzahl von Parametern untereinander kombiniert ist, wobei der genannte Vergleichsschritt (4) im Vergleich eines Ergebnisses besteht, das aus der genannten Kombination von Parametern mit wenigstens einem Referenzparameter stammt.

**3.** Verfahren gemäß irgendeinem der Ansprüche 1 oder 2, **dadurch gekennzeichnet, dass** es einen Generierungsschritt (23) einer elastischen Welle niedriger Frequenz in dem genannten biologischen Gewebe (16) umfasst.

**4.** Verfahren gemäß Anspruch 3, **dadurch gekennzeichnet, dass** die genannte elastische Welle niedriger Frequenz per Vibration eines Generators (11) mit einer Welle niedriger Frequenz generiert wird.

**5.** Verfahren gemäß Anspruch 3, **dadurch gekennzeichnet, dass** die genannte elastische Welle niedriger Frequenz per Strahlungsdruck generiert wird.

**6.** Verfahren gemäß einem der Ansprüche 1 bis 5, **dadurch gekennzeichnet, dass** ein Indikator einen Operator über das genannte Ergebnis des genannten Vergleichsschritts (4) informiert.

**7.** Verfahren gemäß irgendeinem der Ansprüche 1 bis 6, **dadurch gekennzeichnet, dass** der genannte wenigstens eine Parameter und die genannte wenigstens eine Eigenschaft des genannten biologischen Gewebes (16) in Abhängigkeit von Daten bestimmt werden, die aus dem genannten Erwerb (22) des genannten wenigstens einen Ultraschallsignals extrahiert werden, das von dem biologischen Gewebe (16) reflektiert wird.

8. Verfahren gemäß irgendeinem der Ansprüche 1 bis 7, **dadurch gekennzeichnet, dass** die genannte wenigstens eine Eigenschaft des genannten biologischen Gewebes (16) durch die Umsetzung eines Elastographieverfahrens bestimmt wird.

9. Verfahren gemäß irgendeinem der Ansprüche 1 bis 8, **dadurch gekennzeichnet, dass** die genannte wenigstens eine Eigenschaft die Elastizität des genannten biologischen Gewebes (16) ist.

10. Verfahren gemäß irgendeinem der Ansprüche 1 bis 7, **dadurch gekennzeichnet, dass** die wenigstens eine Eigenschaft eine Ultraschallabschwächung des genannten biologischen Gewebes (16) ist.

11. Verfahren gemäß irgendeinem der Ansprüche 1 bis 10, **dadurch gekennzeichnet, dass** der genannte wenigstens eine Parameter ein Ultraschallparameter des genannten biologischen Gewebes (16) ist.

12. Verfahren gemäß Anspruch 11, **dadurch gekennzeichnet, dass** der wenigstens eine Ultraschallparameter eine Ultraschallabschwächung des genannten biologischen Gewebes (16) ist.

13. Verfahren gemäß einem der Ansprüche 1 bis 10, **dadurch gekennzeichnet, dass** der genannte wenigstens eine Parameter ein viskoelastischer Parameter des genannten biologischen Gewebes (16) ist.

14. Verfahren gemäß Anspruch 13, **dadurch gekennzeichnet, dass** der genannte viskoelastische Parameter die Elastizität des genannten biologischen Gewebes (16) ist.

15. Verfahren gemäß Anspruch 14, **dadurch gekennzeichnet, dass** die genannte Elastizität durch ein Elastographieverfahren erhalten wird.

16. Verfahren gemäß Anspruch 1 bis 10, **dadurch gekennzeichnet, dass** der genannte wenigstens eine Parameter ein physiologischer Parameter des genannten biologischen Gewebes (16) ist.

**Claims**

1. Method for measuring at least one property of biological tissue (12) comprising the steps of:

   - positioning (1) an ultrasonic transducer (12) opposite said biological tissue (16) to be measured;
   - generating (21) at least one ultrasonic signal within said biological tissue (16),
   - acquiring (22) at least one ultrasonic signal reflected by said biological tissue (16);
   - determining (3) at least one parameter of said biological tissue (16) by means of said acquisition (22) of said at least one ultrasonic signal reflected by said biological tissue (16), said at least one parameter being representative of said biological tissue (16);

   said method being **characterised in that** it further comprises the steps of

   - comparing (4) said at least one parameter of said biological tissue (16) with at least one reference parameter of a target biological tissue so as to confirm the hypothesis of the presence of said target biological tissue opposite said ultrasonic transducer; said comparison (4) consisting in comparing a value of said at least one determined value with a value of said at least one reference parameter,
   - determining (5) at least one property of said biological tissue (16) on the basis of the result of said comparison step (4), said step of determining (5) said at least one property being carried out only if the absolute value of the difference between said value of said at least one determined parameter and said value of said at least one reference parameter is less than a given threshold value.

2. Method according to claim 1 **characterised in that** a plurality of parameters are combined together, said step of comparing (4) consisting in comparing a result from said combination of parameters with at least one reference parameter.

3. Method according to one of claims 1 or 2 **characterised in that** it comprises a step of generating (23) a low-frequency elastic wave in said biological tissue (16).

4. Method according to claim 3 **characterised in that** said low-frequency elastic wave is generated by vibration of a generator (11) of low-frequency elastic waves.

5. Method according to claim 3 **characterised in that** said low-frequency elastic wave is generated by radiation pressure.

6. Method according to one of claims 1 to 5 **characterised in that** an indicator informs an operator of said result of said step of comparing (4).

7. Method according to one of claims 1 to 6 **characterised in that** said at least one parameter and said at least one property of said biological tissue (16) are determined according to data extracted from said acquisition (22) of said at least one ultrasonic signal reflected by the biological tissue (16).

8. Method according to one of claims 1 to 7 **characterised in that** said at least one property of said biological tissue (16) is determined by the implementation of a method of elastography.

9. Method according to one of claims 1 to 8 **characterised in that** said at least one property is the elasticity of said biological tissue (16).

10. Method according to one of claims 1 to 7 **characterised in that** said at least one property is an ultrasonic attenuation of said biological tissue (16),

11. Method according to one of claims 1 to 10 **characterised in that** said at least one parameter is an ultrasonic parameter of said biological tissue (16).

12. Method according to claim 11 **characterised in that** said at least one ultrasonic parameter is an ultrasonic attenuation of said biological tissue (16).

13. Method according to one of claims 1 to 10 **characterised in that** said at least one parameter is a viscoelastic parameter of said biological tissue (16).

14. Method according to claim 13 **characterised in that** said viscoelastic parameter is the elasticity of said biological tissue (16).

15. Method according to claim 14 **characterised in that** said elasticity is obtained by a method of elastography.

16. Method according to one of claims 1 to 10 **characterised in that** said at least one parameter is a physiological parameter of said biological tissue (16).

-1-

-2-
-21-
-22-
-23-

-3-

-4-

-5-

**Fig. 1**

**Fig. 2**

10
11
12
14
16
13
15
17
X

**Fig. 3**

19
18
20
10
12
16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2843290
- US 6013031 A1

- US 4441368 A